# EUROPÄISCHE PATENTSCHRIFT

(11) **EP 1 320 330 B1**
(45) Veröffentlichungstag und Bekanntmachung des Hinweises auf die Patenterteilung: **04.07.2007**
(21) Anmeldenummer: 00960281.4
(22) Anmeldetag: 27.09.2000
(51) Int. Cl.: A61B 17/16

(54) **VORRICHTUNG ZUR VERBINDUNG CHIRURGISCHER RÄUM-ODER BOHRWERKZEUGBESTANDTEILEN**
DEVICE FOR CONNECTING SURGICAL CLEARING OR DRILLING TOOL COMPONENTS
DISPOSITIF DE CONNEXION D'OUTILS D'EVACUATION OU DE PER AGE CHIRURGICAUX

(43) Veröffentlichungstag der Anmeldung: 25.06.2003
(73) Patentinhaber: Synthes GmbH, 4436 Oberdorf (CH)
(72) Erfinder: STEIGER, Peter, CH-3360 Herzogenbuchsee (CH); BRUNNER, Peter, CH-3074 Muri bei Bern (CH)
(74) Vertreter: Lusuardi, Werther
(86) Internationale Anmeldenummer: PCT/CH2000/000527
(87) Internationale Veröffentlichungsnummer: WO 2002/026141

(56) Entgegenhaltungen:
- EP-A- 0 195 150
- DE-U- 29 713 920
- US-A- 5 693 047
- US-A- 5 720 749

## Beschreibung

Die Erfindung bezieht sich auf eine Vorrichtung zur koaxialen Verbindung von chirurgischen Räum- oder Bohrwerkzeugen mit einer Welle, gemäss dem Oberbegriff des Patentanspruchs 1.

Bei verschiedenen in der Chirurgie eingesetzten Instrumenten ist eine Drehmoment-und Kraftübertragung zwischen zwei Bestandteilen des Instrumentes notwendig. Die Drehmomentübertragung dient beispielsweise zum rotativen Antrieb eines chirurgischen Werkzeuges, während die Kraftübertragung in axialer Richtung erfolgt. Vorteilhaft für Drehmoment- und Kraftübertragung sind formschlüssige Verbindungen zwischen den beiden Teilen. Solche Verbindungen treten beispielsweise bei der Kopplung einer flexiblen Bohrwelle mit den Bohrköpfen für die Aufbohrung von Markräumen in Knochen auf.

Eine solche Vorrichtung mit formschlüssigen Verbindungen für die Drehmoment- und Axialkraftübertragung zwischen einem Antriebsmittel und einer flexiblen Welle sowie zwischen der flexiblen Welle und einem Räumwerkzeug ist in der US 5,720,749 RUPP offenbart. In einer Ausführungsform werden in einem Hohlkörper am Räumwerkzeug, welcher zur axialen Aufnahme der flexiblen Welle dient, vor dem Zusammenfügen von Räumwerkzeug und flexibler Welle durch plastische Verformung Nocken in den zylindrischen Bohrungsabschnitt geformt. Anschliessend wird die flexible Welle in axialer Richtung in diesen so mit Nocken versehenen Bohrungsabschnitt gepresst, wodurch die Welle durch die Nocken radial deformiert wird und somit tangential formschlüssig und axial mittels einem Presssitz mit dem Hohlkörper verbunden ist. Nachteilig an dieser bekannten Verbindung ist, dass durch das Einpressen der flexiblen Welle in die mit Nocken versehene Bohrung des Hohlkörpers die flexible Welle durch die Nocken erheblich deformiert wird und somit trotz der axialen konischen Zentrierung am vorderen Ende keine konzentrische respektive koaxiale Verbindung gewährleistet ist.

Hier will die Erfindung Abhilfe schaffen. Der Erfindung liegt die Aufgabe zugrunde, eine formschlüssige Verbindung für die Drehmoment- und Axialkraftübertragung zu schaffen, bei welcher das Zusammenfügen der flexiblen Welle mit dem Hohlkörper mittels einer Schiebepassung absolut konzentrisch und koaxial erfolgt und eine rotativ und axial formschlüssige Verbindung herstellbar ist.

Die Erfindung löst die gestellte Aufgabe mit einer Vorrichtung zur koaxialen Verbindung von chirurgischen Räum- oder Bohrwerkzeugen mit einer Welle, welche die Merkmale des Anspruchs 1 aufweist.

Die erfindungsgemässe Vorrichtung zur koaxialen Verbindung von chirurgischen Räum-oder Bohrwerkzeugen mit einer Welle umfasst eine Welle und einen Hohlkörper mit einem zur Zentralachse koaxialen Hohlraum, wobei die Welle mit ihrem vorderen Ende vom ersten Ende her koaxial in den Hohlraum eingeführt ist und der Hohlkörper mit seinem zweiten Ende mit einem Instrumenten-Bestandteil, beispielsweise einem Kupplungsteil oder einem Bohrkopf für Markraumbohrungen verbindbar ist. Zudem weist die Wand des Hohlkörpers mindestens eine plastisch verformte Einprägung mit einer Erhebung im Hohlraum auf. Dabei kann die durch die mindestens eine Erhebung hervorgerufene Verformung an der Welle eine plastische und/oder elastische Verformung sein.

In der bevorzugten Ausführungsform der erfindungsgemässen Vorrichtung ist die Welle am vorderen Ende koaxial zur Zentralachse kreiszylindrisch ausgestaltet und weist einen Durchmesser d auf. Der Hohlraum ist ebenfalls koaxial zur Zentralachse kreiszylindrisch ausgebildet und weist einen Durchmesser D auf, wobei die Durchmesser d und D so bemessen sind, dass zwischen Welle und Hohlraum ein Schiebesitz gebildet wird. Durch den Schiebesitz wird eine genau koaxiale Verbindung zwischen Welle und Hohlkörper ermöglicht, welche durch das Aufbringen von Presskräften bei der Herstellung der plastischen Einprägungen in den Hohlkörper nach dem Einschieben der Welle nicht beeinträchtigt wird. Dadurch ist eine feste, axial und tangential formschlüssige Verbindung zwischen Welle und Hohlkörper herstellbar, welche eine konzentrische Rotation der Instrumenten-Bestandteile gewährleistet.

In einer weiteren Ausführungsform der erfindungsgemässen Vorrichtung umfasst der Hohlraum vom ersten Ende her gemessen, in einer Tiefe T eine axiale Anschlagfläche für die Welle. Durch diese axiale Anschlagfläche entsteht zusätzlich zur axial und tangential formschlüssigen Verbindung zwischen Welle und Hohlkörper mittels der Einprägungen eine Sicherung zur Aufnahme von grossen axialen Druckkräften auf die Vorrichtung.

Vorzugsweise beträgt die Wandstärke des Hohlkörpers zwischen 0,1 mm und 1,0 mm während die Erhebungen vorzugsweise zwischen 0,05 mm und 0,7 mm von der Wand vorstehen. -

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung, eine feste Verbindung zwischen der Welle und dem Hohlkörper mit dem Instrumentenbestandteil, beispielsweise einem Kupplungsteil oder einem Bohrkopf für den Markraum herstellbar ist. Die Einzelteile der erfindungsgemässen Vorrichtung sind einfach anzufertigen und die Verbindung zwischen Welle und Hohlkörper, welche durch die plastische Verformung der Wand nach dem Einschieben der Welle in die Wand erfolgt, ist mit einfachsten Mitteln herstellbar. Ferner sind kleinere Wellendurchmesser sowie ein Links-/Rechtslauf der Antriebsmaschine möglich.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Darstellung einer Ausführungsform der erfindungsgemässen Vorrichtung verbunden mit einem chirurgischen Räumwerkzeug;
Fig. 2 einen Schnitt durch Welle und Hohlkörper der in Fig. 1 dargestellten Ausführungsform der erfindungsgemässen Vorrichtung; und
Fig. 3 eine perspektivische Darstellung einer anderen Ausführungsform der erfindungsgemässen Vorrichtung.

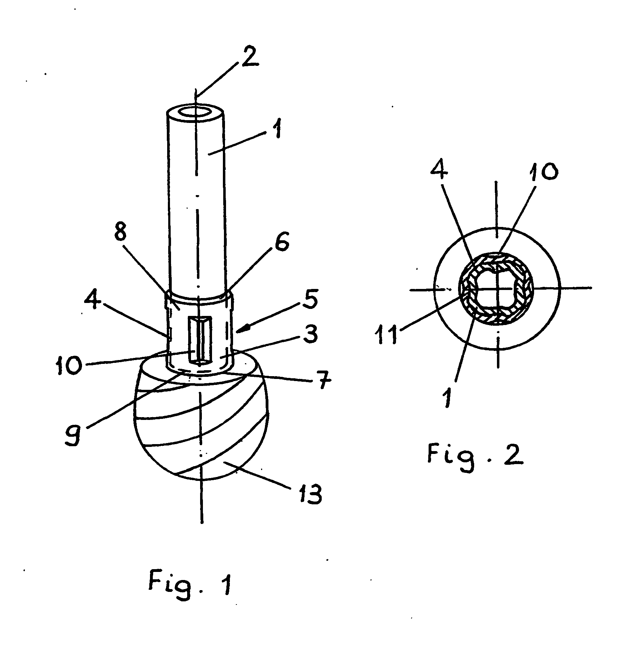

In den Fig. 1 und 2 ist eine Ausführungsform der erfindungsgemässen Vorrichtung dargestellt, welche einen fest mit einem chirurgischen Bohrkopf 13 verbundenen Hohlkörper 5 umfasst. Die am Antrieb (nicht gezeichnet) angekuppelte zylindrische Welle 1 wird koaxial bezüglich der Zentralachse 2 in den zylindrischen Hohlraum 4 im Hohlkörper 5 eingeführt, wobei der Aussendurchmesser der Welle 1 und der Innendurchmesser des Hohlraumes 4 einen Schiebesitz bilden, so dass Welle 1 und Hohlkörper 5 exakt konzentrisch ausgerichtet werden und doch die Welle 1 ohne Kraftaufwand in den Hohlraum 4 einführbar ist. In einer bestimmten Tiefe des Hohlraumes 4 ist quer zur Zentralachse 2 eine axiale Anschlagfläche 9 angebracht, welche beispielsweise mit der hohlkörperseitigen Stirnfläche des Bohrkopfes 13 übereinstimmen kann. Der Hohlkörper 5 ist konzentrisch zur Zentralachse 2 hohlzylindrisch ausgestaltet und umfasst wellenseitig ein erstes Ende 6, bohrkopfseitig ein zweites Ende 7, eine Wand 8 und einen zur Zentralachse 2 koaxialen Hohlraum 4. Nachdem die Welle 1 mit ihrem vorderen, bohrkopfseitigen Ende 3 vom ersten, wellenseitigen Ende 6 des Hohlkörpers her koaxial in den Hohlraum 4 eingeführt wurde, wird mittels eines Prägewerkzeuges (nicht gezeichnet) die Wand 8 des Hohlkörpers 5 so plastisch verformt, dass vier Einprägungen 10 entstehen, welche je eine Erhebung 11 im Hohlraum 4 aufweisen. Durch die Erhebungen 11 wird die Welle 1 an ihrem Umfang entsprechend zu den Erhebungen 11 verformt, so dass bezüglich Rotation um die Zentralachse 2 und bezüglich Verschiebung zwischen Welle 1 und Hohlkörper 5 koaxial zur Zentralachse 2 eine formschlüssige Fixierung hergestellt wird. In der hier gezeigten Ausführungsform der erfindungsgemässen Vorrichtung sind Hohlkörper 5 und Bohrkopf 13 einstückig.

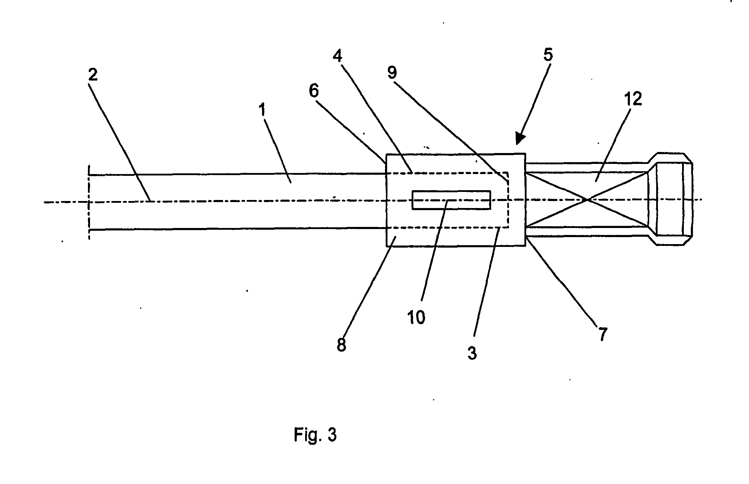

Die in Fig. 3 dargestellte Ausführungsform der erfindungsgemässen Vorrichtung unterscheidet sich von der in Fig. 1 beschriebenen Ausführungsform nur darin, dass der Hohlkörper 5 an seinem zweiten, axial von der Welle 1 entfernten Ende 7 mit einem Kupplungsteil 12 verbunden ist, wobei das Kupplungsteil 12 zur lösbaren Verbindung mit einem chirurgischen Räum- oder Bohrwerkzeug (nicht gezeichnet) dient.

## Patentansprüche

1. Vorrichtung zur koaxialen Verbindung von chirurgischen Räum- und Bohrwerkzeugen mit einer Welle (1), welche
A) eine Welle (1) mit einer Zentralachse (2) und einem vorderen Ende (3):
B) einen Hohlkörper (5) mit einem ersten Ende (6), einem zweiten Ende (7), einer Wand (8) und einem zur Zentralachse (2) koaxialen Hohlraum (4) umfasst, wobei die Welle (1) mit ihrem vorderen Ende (3) vom ersten Ende (6) her koaxial in den Hohlraum (4) eingeführt ist und der Hohlkörper (5) mit seinem zweiten Ende (7) mit einem Werkzeug oder Kupplungsteil verbindbar oder verbunden ist;
C) die Wand (8) mindestens eine eine Erhebung (11) im Hohlraum (4) aufweisende, plastisch verformte Einprägung (10) aufweist und die Welle (1) durch die mindestens eine Erhebung (11) elastisch verformt wird: und
D) die Einprägung (10) eine feste, tangential formschlüssige Verbindung zwischen Welle (1) und Hohlkörper (5) gewährleistet, **dadurch gekennzeichnet, dass** die Einprägung (10) auch eine feste axial formschlüssige Verbindung zwischen Welle (1) und Hohlkörper (5) gewährleistet.

2. Vorrichtung nach Anspruch 1, **dadurch gekennzeichnet, dass** die Welle (1) am vorderen Ende (3) koaxial zur Zentralachse (2) kreiszylindrisch ist und einen Durchmesser d ausweist, dass der Hohlraum (4) koaxial zur Zentralachse (2) kreiszylindrisch ist und einen Durchmesser D aufweist und dass die Durchmesser d und D so bemessen sind, dass zwischen Welle (1) und Hohlraum (4) ein Schiebesitz gebildet wird.

3. Vorrichtung nach Anspruch 1 oder 2, **dadurch gekennzeichnet, dass** die Welle (1) durch die mindestens eine Erhebung (11) elastisch und plastisch verformt wird.

4. Vorrichtung nach Anspruch 1 oder 2, **dadurch gekennzeichnet, dass** die Welle (1) durch die mindestens eine Erhebung (11) plastisch verformt wird.

5. Vorrichtung nach einem der Ansprüche 1 bis 4, **dadurch gekennzeichnet, dass** der Hohlraum (4) vom ersten Ende (6) her in einer Tiefe T eine axiale Anschlagfläche (9) für die Welle (1) aufweist

6. Vorrichtung nach einem der Ansprüche 1 bis 5, **dadurch gekennzeichnet, dass** die Wandstärke des Hohlkörpers (5) zwischen 0,1 mm und 1,0 mm beträgt.

7. Vorrichtung nach einem der Ansprüche 1 bis 6, **dadurch gekennzeichnet**, die mindestens eine Erhebung (11) zwischen 0,05 mm und 0,7 mm von der Wand (8) gegen die Zentralachse (2) vorsteht.

8. Vorrichtung nach einem der Ansprüche 1 bis 7, **dadurch gekennzeichnet, dass** der Hohlkörper (5) mit einem chirurgischen Räum- oder Bohrwerkzeug einstückig ausgebildet ist.

## Claims

1. A device for coaxially connecting surgical reaming or drilling tools with a shaft (1), comprising
A) a shaft (1) with a central axis (2) and a front end portion (3);
B) a hollow body (5) having a first end portion (6), a second end portion (7), a wall (8), and a cavity (4) extending coaxially to the central axis (2), the shaft (1) being inserted into the cavity (4) with the shaft front end portion (3) entering the cavity coaxially from the first end portion (6) of the hollow body (5), said hollow body (5) being connectible, or being connected, with its second end portion (7) to a tool or to a coupling member;
C) the wall (8) is provided with at least one plastically deformed impression (10) having a projection (11) sticking out into the cavity (4) and that the shaft (1) is subject to an elastic deformation by means of the at least one projection (11); and
D) the impression (10) ensures a firm tangentially positive connection between the shaft (1) and the hollow body (5),
**characterized in that** the impression (10) also ensures a firm axially positive connection between the shaft (1) and the hollow body (5).

2. A device as claimed in claim 1, **characterised in that** on its front end portion (3), the shaft (1) is shaped in the form of a regular cylinder extending coaxially to the central axis (2) and has a diameter d, **in that** the cavity (4) is shaped in the form of a regular cylinder extending coaxially to the central axis (2) and has a diameter D, and **in that** the diameters d and D are dimensioned in such a way that a close-sliding fit is formed between the shaft (1) and the cavity (4).

3. A device as claimed in claim 1 or 2, **characterised in that** the shaft (1) is subjected to elastic and plastic deformation by means of the at least one projection (11).

4. A device as claimed in claim 1 or 2, **characterised in that** the shaft (1) is subjected to plastic deformation by means of the at least one projection (11).

5. A device as claimed in any of the claims 1 to 4, **characterised in that** the cavity (4) has an axial stop surface (9) formed at a depth T as measured from the first end portion (6) for the shaft (1) to rest upon.

6. A device as claimed in any of the claims 1 to 5, **characterised in that** the wall thickness of the hollow body (5) is comprised between 0.1 and 1.0 mm.

7. A device as claimed in any of the claims 1 to 6, **characterised in that** the at least one projection (11) protrudes from the wall (8) in the direction of the central axis (2) by an amount of between 0.05 mm and 0.7 mm.

8. A device as claimed in any of the claims 1 to 7, **characterised in that** the hollow body (5) is formed in a single piece with a surgical reaming or drilling tool.

## Revendications

1. Dispositif pour relier de manière coaxiale des outils chirurgicaux de brochage et d'alésage à un arbre (1), comprenant
A) un arbre (1) ayant un axe central (2) et une extrémité avant (3) ;
B) un corps creux (5) ayant une première extrémité (6), une deuxième extrémité (7), une paroi (8) et un espace creux (4) coaxial à l'axe central (2), dans lequel l'arbre (1) est inséré coaxialement dans l'espace creux (4) avec son extrémité avant (3), vue depuis la première extrémité (6), et l'espace creux (5) est relié ou peut être relié à un outil ou à un élément d'accouplement avec sa deuxième extrémité (7) ;
C) la paroi (8) présentant au moins un estampage (10) obtenu par déformation plastique présentant un bossage (11) dans l'espace creux (4), et l'arbre (1) étant déformé de manière élastique par le au moins un bossage (11) ; et
D) l'estampage (10) garantissant une liaison par emboîtement tangentiel solide entre l'arbre (1) et le corps creux (5),
**caractérisé en ce que** l'estampage (10) garantit également une liaison par emboîtement axial solide entre l'arbre (1) et le corps creux (5).

2. Dispositif selon la revendication 1, **caractérisé en ce que** l'arbre (1), au niveau de son extrémité avant (3), coaxialement à l'axe central (2), a la forme d'un cylindre circulaire et présente un diamètre d, **en ce que** l'espace creux (4), coaxialement à l'axe central (2), a la forme d'un cylindre circulaire et présente un diamètre D, et **en ce que** les diamètres d et D sont dimensionnés de manière à obtenir un ajustement appuyé entre l'arbre (1) et l'espace creux (4).

3. Dispositif selon la revendication 1 ou 2, **caractérisé en ce que** l'arbre (1) est déformé de manière élastique et plastique par le au moins un bossage (11).

4. Dispositif selon la revendication 1 ou 2, **caractérisé en ce que** l'arbre (1) est déformé de manière plastique par le au moins un bossage (11).

5. Dispositif selon l'une quelconque des revendications 1 à 4, **caractérisé en ce qu'**à une profondeur T, vue depuis la première extrémité (6), l'espace creux (4) présente une surface d'arrêt axiale (9) pour l'arbre (1).

6. Dispositif selon l'une quelconque des revendications 1 à 5, **caractérisé en ce que** l'épaisseur de paroi du corps creux (5) est comprise entre 0,1 mm et 1,0 mm.

7. Dispositif selon l'une quelconque des revendications 1 à 6, **caractérisé en ce que** le au moins un bossage (11) dépasse de la paroi (8) en direction de l'axe central (2) d'une distance comprise entre 0,05 mm et 0,7 mm.

8. Dispositif selon l'une quelconque des revendications 1 à 7, **caractérisé en ce que** le corps creux (5) est formé d'une seule pièce avec un outil chirurgical de brochage et d'alésage.
